# FASCICULE DE BREVET EUROPEEN

(11) **EP 1 663 285 B1**
(45) Date de publication et mention de la délivrance du brevet: **16.01.2013**
(21) Numéro de dépôt: 04767703.4
(22) Date de dépôt: 16.07.2004
(51) Int. Cl.: A61K 38/07, A61K 38/08, A61P 17/14, A61K 8/64, A23L 1/305, A61Q 7/00

(54) **UTILISATION DE CONJUGUES PEPTIDIQUES POUR LA PREPARATION DE COMPOSITIONS DESTINEES AU TRAITEMENT PREVENTIF ET CURATIF DEL'ALOPECIE**
VERWENDUNG VON PEPTIDISCHEN KONJUGATEN ZUR HERSTELLUNG VON ZUSAMMENSETZUNGEN FÜR DIE PRÄVENTIVE UND KURATIVE BEHANDLUNG VON ALOPEZIE
USE OF PEPTIDIC CONJUGATES FOR PREPARING COMPOSITIONS FOR ALOPECIA PREVENTIVE AND CURATIVE TREATMENT

(30) Priorité: 18.07.2003 FR 0308801
(43) Date de publication de la demande: 07.06.2006
(73) Titulaire: INSTITUT EUROPEEN DE BIOLOGIE CELLULAIRE, 31520 Ramonville Saint-Agne (FR)
(72) Inventeur: PINEL, Anne-Marie, Résidence Le Galoubet, F-31400 Toulouse (FR); HOCQUAUX, Michel, F-75012 Paris (FR)
(74) Mandataire: Regimbeau
(86) Numéro de dépôt international: PCT/FR2004/001879
(87) Numéro de publication internationale: WO 2005/009456

(56) Documents cités:
- WO-A-00/58347
- WO-A-91/07431
- WO-A-97/18235
- DE-A- 4 127 790
- BECK-PIOTRASCHKE K ET AL: "Protease-catalysed synthesis of peptides containing histidine and lysine" TETRAHEDRON: ASYMMETRY, ELSEVIER SCIENCE PUBLISHERS, AMSTERDAM, NL, vol. 9, no. 9, 8 mai 1998 (1998-05-08), pages 1505-1518, XP004131205 ISSN: 0957-4166
- PICKART L: "THE USE OF GLYCYLHISTIDYLLYSINE IN CULTURE SYSTEMS" IN VITRO, TISSUE CULTURE ASSOCIATION, US, vol. 17, no. 6, juin 1981 (1981-06), pages 459-466, XP009012941 ISSN: 0073-5655

### LISTE DE SEQUENCES

<110> INSTITUT EUROPEEN DE BIOLOGIE CELLULAIRE
<120> UTILISATION DE CONJUGUES PEPTIDIQUES POUR LA PREPARATION DE COMPOSITIONS DESTINEES AU TRAITEMENT PREVENTIF ET CURATIF DE L'ALOPECIE
<130> D21280
<150> FR 03/08 801 <151> 2003-07-18
<160> 18
<170> PatentIn version 3.2
<210> 1
   <211> 4
   <212> PRT
   <213> Séquence artificielle
<220>
   <223> Peptide
<220>
   <221> misc_feature
   <222> (1) .. (1)
   <223> Xaa peut être 1 à trois résidus Lys ou MeLys
<400> 1
   Xaa Gly His Lys
   1
<210> 2
   <211> 4
   <212> PRT
   <213> Séquence artificielle
<220>
   <223> Peptide
<220>
   <221> misc feature
   <222> (1).. (1)
   <223> Xaa peut être 1 à trois résidus Lys ou MeLys
<220>
   <221> MOD_RES
   <222> (4)..(4)
   <223> AMIDATION
<400> 2
   Xaa Gly His Lys
   1
<210> 3
   <211> 4
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa peut être 1 à trois résidus Lys, MeLys ou une liaison.
<400> 3
   Xaa Gly His Lys
   1
<210> 4
   <211> 4
   <212> PRT
   <213> Séquence artificielle
<220>
   <223> Peptide
<220>
   <221> MOD_RES
   <222> (1) .. (1)
   <223> BLOCKED
<220>
   <221> misc_feature
   <222> (1)..(1)
   <223> Xaa peut être 1 à trois résidus Lys, MeLys ou une liaison.
<220>
   <221> MOD_RES
   <222> (4)..(4)
   <223> AMIDATION
<400> 4
   Xaa Gly His Lys
   1
<210> 5
   <211> 6
   <212> PRT
   <213> Séquence artificielle
<220>
   <223> Peptide
<220>
   <221> MOD_RES
   <222> (1) .. (1)
   <223> BLOCKED
<220>
   <221> misc_feature
   <222> (1)..(1)
   <223> Xaa = MeLys
<220>
   <221> MOD_RES
   <222> (6)..(6)
   <223> AMIDATION
<400> 5
   Xaa Lys Lys Gly His Lys
   1               5
<210> 6
   <211> 5
   <212> PRT
   <213> Séquence artificielle
<220>
   <223> Peptide
<220>
   <221> MOD_RES
   <222> (1)..(1)
   <223> BLOCKED '
<220>
   <221> misc_feature
   <222> (1)..(1)
   <223> Xaa = MeLys
<220>
   <221> MOD_RES
   <222> (5)..(5)
   <223> AMIDATION
<400> 6
   Xaa Lys Gly His Lys
   1               5
<210> 7
   <211> 4
   <212> PRT
   <213> Séquence artificielle
<220>
   <223> Peptide
<220>
   <221> MOD_RES
   <222> (1)..(1)
   <223> BLOCKED
<220>
   <221> mise feature
   <222> (1)..(1)
   <223> Xaa = MeLys
<220>
   <221> MOD_RES
   <222> (4)..(4)
   <223> AMIDATION
<400> 7
   Xaa Gly His Lys
   1
<210> 8
   <211> 6
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa = MeLys
<400> 8
   Xaa Lys Lys Gly His Lys
   1               5
<210> 9
   <211> 5
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa = MeLys
<400> 9
   Xaa Lys Gly His Lys
   1               5
<210> 10
   <211> 4
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa = MeLys
<400> 10
   Xaa Gly His Lys
   1
<210> 11
   <211> 5
   <212> PRT
   <213> Séquence artificielle
<220>
   <223> Peptide
<220>
   <221> MOD_RES
   <222> (1)..(1)
   <223> BLOCKED
<220>
   <221> MOD_RES
   <222> (5)..(5)
   <223> AMIDATION
<400> 11
   Lys Lys Gly His Lys
   1 5
<210> 12
   <211> 4
   <212> PRT
   <213> Séquence artificielle
<220>
   <223> Peptide
<220>
   <221> MOD_RES
   <222> (1)..(1)
   <223> BLOCKED
<220>
   <221> MOD_RES
   <222> (4)..(4)
   <223> AMIDATION
<400> 12
   Lys Gly His Lys
   1
<210> 13
   <211> 5
   <212> PRT
   <213> Séquence artificielle
<220>
   <223> Peptide
<220>
   <221> MOD_RES
   <222> (1)..(1)
   <223> BLOCKED
<400> 13
   Lys Lys Gly His Lys
   1 5
<210> 14
   <211> 4
   <212> PRT
   <213> Séquence artificielle
<220>
   <223> Peptide
<220>
   <221> MOD_RES
   <222> (1)..(1)
   <223> BLOCKED
<400> 14
   Lys Gly His Lys
   1
<210> 15
   <211> 5
   <212> PRT
   <213> Séquence artificielle
<220>
   <223> Peptide
<220>
   <221> misc_feature
   <222> (1)..(1)
   <223> Xaa peut être les séquences Glu-Gln-Arg, Arg-Lys, Arg-Lys-Asp ou l'acide aminé Arg ou une liaison
<220>
   <221> misc_feature
   <222> (5)..(5)
   <223> Xaa Peut être les séquences Tyr-Val-Gln-Leu-Tyr-Amidée, Leu-DOPA, les acides aminés Dopa amidé ou HomoPhe amidé
<400> 15
   Xaa Lys Asp Val Xaa
   1               5
<210> 16
   <211> 5
   <212> PRT
   <213> Séquence artificielle
<220>
   <223> Peptide
<220>
   <221> misc_feature
   <222> (1)..(1)
   <223> Xaa Peut être les séquences Gly-Gln-Gln ou Glu-Gln
<220>
   <221> misc_feature
   <222> (5)..(5)
   <223> Xaa Peut être les séquences Tyr-Val-Gln-Leu-Tyr-Amidée, Leu-DOPA, Val-Tyr, Val-Tyr-amidé, ou les acides aminés Tyr, Tyr amidé, Dopa amidé ou HomoPhe amidé
<400> 16
   Xaa Lys Asp Val Xaa
   1               5
<210> 17
   <211> 5
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa peut être les séquences Glu-Gln-Arg, Arg-Lys, Arg-Lys-Asp ou l'acide aminé Arg ou une liaison
<220>
   <221> misc_feature
   <222> (5)..(5)
   <223> Xaa Peut être les séquences Tyr-Val-Gln-Leu-Tyr-Amidée, Leu-DOPA, les acides aminés Dopa amidé ou HomoPhe amidé
<400> 17
   Xaa Lys Asp Val Xaa
   1               5
<210> 18
   <211> 5
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa Peut être les séquences Gly-Gln-Gln ou Glu-Gln
<220>
   <221> misc_feature
   <222> (5)..(5)
   <223> Xaa Peut être les séquences Tyr-Val-Gln-Leu-Tyr-Amidée, Leu-DOPA, Val-Tyr, Val-Tyr-amidé, ou les acides aminés Tyr, Tyr amidé, Dopa amidé ou HomoPhe amidé
<400> 18
   Xaa Lys Asp Val Xaa
   1               5

## Description

L'invention se rapporte à l'utilisation de conjugués peptidiques contenant la séquence Gly-His-Lys pour la préparation de compositions dermatologiques ou cosmétologiques pour stimuler la croissance du cheveux ou freiner sa chute.

Durant toute la vie d'un individu, la croissance des cheveux et leur renouvellement sont déterminés par l'activité des follicules pileux. Ils effectuent un cycle régulier composé de trois phases : anagène, catagène et télogène, qui sont chacune caractérisées par des mécanismes moléculaires et cellulaires bien précis :
- Durant la phase anagène qui dure environ trois ans, les cellules de la papille dermique "envoient" des signaux aux cellules souches présentes dans le bulbe. Les cellules compétentes qui reçoivent ces signaux migrent alors vers la matrice du follicule pileux, on parle alors de cellules matricielles. Dans cette zone, les cellules de la papille dermique émettent des signaux supplémentaires qui permettent aux cellules matricielles de proliférer dans un premier temps puis de se différencier ce qui permet l'allongement de la tige pilaire. Lors de cette phase, le follicule pileux migre au travers du derme pour se retrouver en anagène VI ancré dans l'hypoderme au contact du tissu adipeux.
- La phase qui suit, dite catagène, est une phase courte qui dure environ trois semaines durant lesquelles les cellules de la partie inférieure du follicule pileux rentrent en apoptose permettant ainsi la dégénérescence du follicule pileux.
- La phase restante, dite télogène, est une phase de latence caractérisée par l'inactivité du follicule pileux durant trois mois et la chute du cheveu avant une nouvelle entrée en phase anagène.

L'apparence étant à notre époque un facteur social primordial, la perte de cheveux est un réel problème qui peut être vécu comme un handicap social par certaines personnes. Chez l'homme, il s'agit dans la plupart des cas d'alopécie androgénique. Ce type d'alopécie est donc dû à un défaut de catabolisme des androgènes et plus précisément de la testostérone au niveau du follicule pileux par les cellules de papille dermique. En effet, il y a accumulation d'un métabolite de la testostérone, la DHT (métabolite qui est produit par action de la 5α - réductase sur la testostérone), au niveau des follicules pileux. Dans un processus normal, ce composé est dégradé puis éliminé dans les urines. A l'heure actuelle, les inhibiteurs de 5α -réductase sont utilisés dans ce type d'alopécie pour ralentir la chute des cheveux.

L'ensemble des connaissances actuelles concernant la biologie du cheveu et du cuir chevelu, les alopécies et les affections du cuir chevelu, leurs traitements sont rassemblées dans : "Pathologie du cheveu et du cuir chevelu" P. Bouhanna et P. Reygagne - Editions Masson.

On recherche depuis de nombreuses années, dans l'industrie cosmétique ou pharmaceutique, des substances permettant de supprimer ou de réduire l'effet de l'alopécie et notamment d'induire ou de stimuler la croissance des cheveux ou de diminuer leur chute.

Un certain nombre de composés sont déjà utilisés comme le minoxidil, le finastéride.

Certains peptides sont connus pour leur action stimulante de la pousse du cheveu toutefois aucun document ne divulgue que les conjugués peptidiques décrits ci-après sont utiles dans le traitement préventif et curatif de l'alopécie.

La présente invention a donc pour objet l'utilisation d'un peptide répondant à la formule générale (I)
X-Gly-His-Lys-Y (I) (SEQ ID N°1-2)
ou un conjugué répondant à la formule générale (II)
A-X-Gly-His-Lys-Y (II) (SEQ ID N°3-4)
dans lesquelles
A représente le radical correspondant à
- un acide monocarboxylique de formule générale (III)

   HOOC-R (III)
dans laquelle
R représente un radical aliphatique en C1-C24, linéaire ou ramifié, éventuellement substitué par un groupe hydroxy, pouvant comporter une ou plusieurs insaturations, avantageusement de 1 à 6,
- l'acide lipoique ou sa forme réduite, l'acide dihydrolipoïque, la N-lipoyl-lysine ou encore l'acide rétinoïque,
X représente une chaîne de 1 à 3 résidus Lys, éventuellement méthylés ,
Y représente un groupe -OH ou -NH2,
les acides aminés étant sous forme D, L ou DL,
pour la préparation d'une composition dermatologique destinée au traitement préventif et curatif des alopécies.

Avantageusement, la séquence peptidique est conjuguée chimiquement ou physiquement avec les acides A. Les peptides conjugués selon l'invention sont liés sous forme de sels, d'esters ou d'amides à ces acides A, la fraction acide carboxilique de l'acide assurant la liaison.

Les acides aminés dans le peptide de formule (I) ou le conjugué peptidique de formule (II) peuvent avoir une configuration D, L ou DL.

Autrement dit, les peptides de formule (I) et les conjugués peptidiques de formule (II) peuvent comporter un ou plusieurs atomes de carbone asymétriques. Ils peuvent donc exister sous forme d'énantiomères ou de diasteréoisomères. Ces énantiomères, diastéréoisomères, ainsi que leurs mélanges, y compris les mélanges racémiques font partie de l'invention.

Les conjugués peptidiques de formule (II) sont des dérivés de faible poids moléculaire qui sont obtenus sous forme d'amides du composé de formule (III).

De plus, les peptides de formule (I) et les conjugués peptidiques de formule (II) peuvent être couplés avec du zinc, sous forme de sel, pour former des complexes.

Les peptides et leurs conjugués peptidiques, ainsi que leur synthèse, sont décrits dans le brevet européen EP 869 969. Ils y sont décrits comme étant utiles dans le traitement en application par voie topique de la cicatrisation des plaies chroniques, la cicatrisation esthétique des plaies chirurgicales, le traitement préventif et curatif des vergetures et de leurs complications. Leur utilisation dans le domaine de la cosmétologie, notamment le traitement préventif et curatif des rides du visage, du cou et des mains, y est également divulguée.

Dans le cadre de la présente invention, on entend par :
- Lys, la lysine, ou un dérivé halogéné de la lysine, tel que la dihydrobromo-méthyl-lysine,
- MeLys, la méthyl-lysine (méthylation en position 6),
- His, l'histidine,
- Gly, la glycine ou un de ses dérivés alkylés, tel que la méthyl-glycine.

Il est également précisé que les peptides de formule (I) ou les conjugués peptidiques de formule (II) mentionnés ci-dessus et dont l'utilisation est l'objet de la présente invention, peuvent être obtenus sous la forme terminale NH2 (autrement dit présentant une fonction amide) et sous la forme terminale OH (autrement dit présentant une fonction acide carboxylique).

De préférence, l'acide de formule (III) est un acide gras polyinsaturé, c'est-à-dire comportant de 1 à 6 insaturations. De manière encore plus préférée, il s'agit d'un acide oméga-3.

Parmi ces acides oméga-3 on peut notamment citer l'acide α -linolénique, l'acide cervonique, l'acide timnodonique et l'acide pinolénique. Les acides cervonique, timnodonique et pinolénique sont également connus sous les dénominations respectives d'acide 4,7,10,13,16,19- docosahexaenoïque (DHA), d'acide 5,8,11,14,17- eicosapentaénoique (EPA) et d'acide 5,9,12-octodécatriénoïque.

Lorsque A représente un acide monocarboxylique de formule générale (III), il peut être avantageusement choisi parmi l'acide acétique, l'acide myristique, l'acide palmitique, les acides hydroxydécénoiques et décénoïques et notamment, l'acide trans-10-hydroxy-Δ2-décénoïque et l'acide trans-oxo-9-decene-2-oïque.

Parmi les conjugués peptidiques de l'invention on peut citer les conjugués peptidiques suivants :
1- A-MeLys-Lys-Lys-Gly-His-Lys-NH2 (SEQ ID N°5),
2- A-MeLys-Lys-Gly-His-Lys-NH2 (SEQ ID N°6),
3- A-MeLys-Gly-His-Lys-NH2 (SEQ ID N°7),
4- A-MeLys-Lys-Lys-Gly-His-Lys-OH (SEQ ID N°8),
5- A-MeLys-Lys-Gly-His-Lys-OH (SEQ ID N°9),
6- A-MeLys-Gly-His-Lys-OH (SEQ ID N°10),
7- A-Lys-Lys-Gly-His-Lys-NH2 (SEQ ID N°11),
8- A-Lys-Gly-His-Lys-NH2 (SEQ ID N°12),
9- A-Lys-Lys-Gly-His-Lys-OH (SEQ ID N°13),
10- A-Lys-Gly-His-Lys-OH (SEQ ID N°14).

Les conjugués peptidiques pour lesquels A est choisi parmi l'acide lipoique et l'acide acétique sont tout particulièrement adaptés, dans le cadre de la présente invention.

On peut encore citer les conjugués peptidiques suivants :
peptide R H-Gly-His-Lys-OH (exemple de réference, non revendiqué)
peptide S Lipoyl-Lys-Gly-His-Lys-NH2,
peptide V Ac-Lys-Gly-His-Lys-NH2.

Les peptides ou leurs conjugués peptidiques peuvent être administrés pour leur utilisation cosmétique par voie topique.

Ils sont préférentiellement administrés par voie topique.

Le conjugué peptidique peut être présent, dans une composition cosmétique topique, à une concentration comprise entre 10⁻⁸ et 10⁻³ M, de préférence entre 10⁻⁷ et 10⁻⁵ M.

La composition cosmétique ou dermatologique peut par exemple se présenter sous forme d'une lotion, d'un shampoing traitant, d'un spray, d'un gel ou d'une crème traitante.

Un autre objet de la présente invention concerne la méthode de traitement cosmétique pour lutter contre la chute des cheveux comprenant l'application sur le cuir chevelu d'une composition comprenant un conjugué peptidique décrit ci-dessus, éventuellement en association comme décrit ci-après.

Ils peuvent être administrés seuls ou en association avec des composés améliorant encore l'activité sur la repousse et ayant déjà été décrits pour cette activité. Parmi ces composés on peut citer :
- le minoxidil,
- des esters de l'acide nicotinique,
- des agents anti-inflammatoires, plus particulièrement de peptides à activité anti-inflammatoire,
- l'acide rétinoïque, ses dérivés et le rétinol,
- des inhibiteurs de la 5α -réductase.

D'autres peptides ou conjugués peptidiques peuvent encore être associés aux peptides ou conjugués peptidiques dont l'utilisation est l'objet de la présente invention. Ils répondent aux formules
W-Lys-Asp-Val-Z (I) (SEQ ID N°15-16)
ou son conjugué peptidique répondant à la formule (II)
A-W-Lys-Asp-Val-Z (II) (SEQ ID N°17-18) dans lesquelles A a la même définition que celle donnée ci-dessus,
et W représente
Glu-Gln-Arg, Arg-Lys, Arg-Lys-Asp, Arg ou une liaison, lorsque Z représente
Tyr-Val-Gln-Leu-Tyr-NH2, Leu-DOPA, DOPA-NH2 ou HomoPhe-NH2,
ou bien W représente
Gly-Gln-Gln ou Glu-Gln,
lorsque Z représente
Tyr-Val-Gln-Leu-Tyr-NH2, Leu-DOPA, Val-Tyr-OH, Val-Tyr-NH2, Tyr-NH2, Tyr-OH, DOPA-NH2 ou HomoPhe-NH2, sous forme d'énantiomères ou de diasteréoisomères, ainsi que leurs mélanges, y compris les mélanges racémiques et les complexes avec le zinc qui peuvent être formés avec ces peptides ou conjugués peptidiques.

On entend par DOPA la dihydroxyphénylalanine et par HomoPhe l'homophénylalanine.

Enfin, un ou plusieurs filtres UVB peuvent encore être associés aux peptides ou aux conjugués peptidiques dont l'utilisation est l'objet de la présente invention, lorsqu'il s'agit d'une administration par voie topique. Ils permettent la photoprotection du cuir chevelu. Ainsi, parmi les filtres UVB adaptés on peut citer, sous leur nom INCI :
- L'acide p-aminobenzoique ou PABA et ses esters :
   *EthylhexyldimethylPABA
   *PEG-25PABA
- Les cinnamates :
   *Ethylhexyl Methoxycinnamate
   *Isoamyl p-Methoxycinnamate
   *Octocrylene
- les Salicylates :
   *Homosalate
   *Ethylhexyl Salicylate
- les benzimidazoles :
   *Phenylbenzimidazole Sulfonic Acid
- les dérivés Benzylidène camphres
   *4-Methylbenzylidene Camphor
   *Benzilidene Camphor
   *Camphor Benzalkonium Methosulfate *Polyacrylamidomethyl Benzylidene Camphor
- les triazines :
   *Ethylhexyl Triazone
   *Diethylhexyl Butamido Triazone.

Les peptides et conjugués peptidiques dont l'utilisation est l'objet de l'invention ont fait l'objet d'essais pharmacologiques permettant de montrer leur activité anti-chute des cheveux.

### Effets des différents peptides sur la croissance de vibrisses de souris in vitro

Afin de montrer l'effet stimulateur des peptides sur la croissance pilaire, des follicules pileux en phase anagène de vibrisses de souris, sont mis en culture selon la technique décrite par Philpott (Philpott et col. 1994. Human Hair gowth in vitro : a model for the study of hair biology. Journal of dermatological science 7 : S55-S72). La croissance de la tige pilaire de follicules pileux a été suivie durant plusieurs jours (JO à J4). Les résultats son reportés dans le tableau ci-après pour les peptides R, S et ' décrits ci-dessus. Ces résultats montrent que ces peptide; stimulent la croissance pilaire lorsque les follicules pileu: sont maintenus en survie *in vitro.*

| | témoin | Peptide R 10⁻⁷M | Peptide S 10⁻⁷M | Peptide V 10⁻⁷M |
|---|---|---|---|---|
| JO | 0,00 | 0,00 | 0,00 | 0,00 |
| J1 | 0,3 | 0,8 | 0,77 | 0,86 |
| J2 | 0,4 | 1,27 | 1,47 | 1,34 |
| J3 | 0,5 | 1,38 | 1,72 | 1,65 |
| J4 | 0,5 | 1,38 | 1,86 | 1,65 |

Les exemples de formulation suivants illustrent la présente invention.

### Exemple 1 : lotion comprenant le conjugué peptidique Ac-Lys-Gly-His-Lys-NH2

(en g)
- Peptide Ac-Lys-Gly-His-Lys-NH2 5.10⁻⁶
- Ethanol à 95° 60
- Propylène glycol 10
- Eau - conservateurs - parfum qsp 100

### Exemple 2 : lotion comprenant le conjugué peptidique Lipoyl-Lys-Gly-His-Lys-NH2

(en g)
- Peptide Lipoyl-Lys-Gly-His-Lys-NH2 10⁻⁵
- Eau 81
- Keltrol T 0,5
- Techpolymer MB-4C 1
- Sepigel 305 0,5
- Huile de Silicone 0,2 1401 2
- Butylène glycol 5

## Revendications

1. Composition dermatologique comprenant un peptide répondant à la formule générale (I)
X-Gly-His-Lys-Y (I) (SEQ ID N°1-2)
ou un conjugué répondant à la formule générale (II)
A-X-Gly-His-Lys-Y (II) (SEQ ID N°3-4)
dans lesquelles
A représente le radical correspondant à
- un acide monocarboxylique de formule générale (III)
HOOC-R (III)
dans laquelle
R représente un radical aliphatique en C1-C24, linéaire ou ramifié, éventuellement substitué par un groupe hydroxy, pouvant comporter une ou plusieurs insaturations, avantageusement de 1 à 6 insaturations,
- l'acide lipoïque ou sa forme réduite, l'acide dihydrolipoïque, la N-lipoyl-lysine ou encore l'acide rétinoïque,
X représente une chaîne de 1 à 3 résidus Lys, éventuellement méthylés,
Y représente un groupe -OH ou -NH2,
les acides aminés étant sous forme D, L ou DL,
sous forme d'énantiomères ou de diasteréoisomères, ainsi que leurs mélanges, y compris les mélanges racémiques, pour utilisation dans le traitement préventif et curatif des alopécies.

2. Composition dermatologique pour utilisation selon la revendication 1, **caractérisé en ce que** l'acide de formule générale (III) est un acide oméga-3 choisi parmi l'acide α -linolénique, l'acide cervonique, l'acide timnodonique et l'acide pinolénique ou bien un radical aliphatique en C1-C24 choisi parmi l'acide acétique, l'acide myristique, l'acide palmitique, les acides hydroxydécénoïques et décénoïques et notamment, l'acide trans-10-hydroxy-Δ2-décénoïque et l'acide trans-oxo-9-decene-2-oïque ou bien un acide choisi parmi l'acide lipoïque ou sa forme réduite, l'acide dihydrolipoïque, la N-lipoyl-lysine ou encore l'acide rétinoïque.

3. Composition dermatologique pour utilisation selon la revendication 1 ou 2, **caractérisé en ce que** A est choisi parmi l'acide lipoïque et l'acide acétique.

4. Composition dermatologique pour utilisation selon l'une quelconque des revendications 1 à 3, **caractérisé en ce que** le conjugué est choisi parmi
1- A-MeLys-Lys-Lys-Gly-His-Lys-NH2 (SEQ ID N°5)
2- A-MeLys-Lys-Gly-His-Lys-NH2 (SEQ ID N°6)
3- A-MeLys-Gly-His-Lys-NH2 (SEQ ID N°7)
4- A-MeLys-Lys-Lys-Gly-His-Lys-OH (SEQ ID N°8)
5- A-MeLys-Lys-Gly-His-Lys-OH (SEQ ID N°9)
6- A-MeLys-Gly-His-Lys-OH (SEQ ID N°10)
7- A-Lys-Lys-Gly-His-Lys-NH2 (SEQ ID N°11)
8- A-Lys-Gly-His-Lys-NH2 (SEQ ID N°12)
9- A-Lys-Lys-Gly-His-Lys-OH (SEQ ID N°13)
10- A-Lys-Gly-His-Lys-OH (SEQ ID N°14)
A étant un acide de formule générale (III) telle que définie dans l'une quelconque des revendications 1 à 3.

5. Composition dermatologique pour utilisation selon l'une quelconque des revendications 1 à 4, **caractérisé en ce que** le peptide ou conjugué peptidique est choisi parmi
Lipoyl-Lys-Gly-His-Lys-NH2
Ac-Lys-Gly-His-Lys-NH2

6. Composition dermatologique pour utilisation selon l'une quelconque des revendications 1 à 5, **caractérisé en ce que** l'on associe au peptide ou au conjugué peptidique un composé améliorant la repousse des cheveux choisi parmi le minoxidil, des esters de l'acide nicotinique, des agents anti-inflammatoires, de l'acide rétinoïque ou ses dérivés, du rétinol ou des inhibiteurs de la 5α-réductase.

7. Composition dermatologique pour utilisation selon l'une quelconque des revendications 1 à 5, **caractérisé en ce que** l'on associe au peptide ou au conjugué un autre peptide répondant à la formule
W-Lys-Asp-Val-Z (I) (SEQ ID N°15-16) ou
son conjugué peptidique répondant à la formule (II)
A-W-Lys-Asp-Val-Z (II) (SEQ ID N°17-18)
dans lesquelles
A a la même définition que celle donnée dans l'une quelconque des revendications 1 à 3,
et W représente
Glu-Gln-Arg, Arg-Lys, Arg-Lys-Asp, Arg ou une liaison, lorsque Z représente
Tyr-Val-Gln-Leu-Tyr-NH2, Leu-DOPA, DOPA-NH2 ou HomoPhe-NH2,
ou bien W représente
Gly-Gln-Gln ou Glu-Gln,
lorsque Z représente
Tyr-Val-Gln-Leu-Tyr-NH2, Leu-DOPA, Val-Tyr-OH, Val-Tyr-NH2, Tyr-NH2, Tyr-OH, DOPA-NH2 ou HomoPhe-NH2,
sous forme d'énantiomères ou de diasteréoisomères, ainsi que leurs mélanges, y compris les mélanges racémiques et les complexes avec le zinc qui peuvent être formés avec ces peptides ou conjugués peptidiques.

8. Composition dermatologique pour utilisation selon l'une quelconque des revendications 1 à 7, **caractérisé en ce que** la composition dermatologique est destinée à l'application topique et **en ce que** l'on associe en outre un filtre UVB choisi parmi l'acide p-aminobenzoïque ou PABA et ses esters tels que l'EthylhexyldimethylPABA ou le PEG-25PABA ; les cinnamates tels que l'Ethylhexyl Methoxycinnamate, l'Isoamyl p-Methoxycinnamate, l'Octocrylene ; les Salicylates tels que l'Homosalate, l'Ethylhexyl Salicylate ; les benzimidazoles tels que le Phenylbenzimidazole Sulfonic Acid ; les dérivés benzylidène camphres tels que le 4-Methylbenzylidene Camphor, le Benzilidene Camphor, le Camphor Benzalkonium Methosulfate et le Polyacrylamidomethyl Benzylidene Camphor et les triazines telles que l'Ethylhexyl Triazone et le Diethylhexyl Butamido Triazone.

9. Méthode de traitement cosmétique pour lutter contre la chute des cheveux comprenant l'application sur le cuir chevelu d'une composition comprenant un peptide ou un conjugué peptidique tel que décrit dans l'une quelconque des revendications 1 à 5, éventuellement associé à un composé améliorant la repousse des cheveux tel que défini dans les revendications 6 et 7 et à un filtre UVB tel que défini dans la revendication 8.

10. Conjugué peptidique de formule lipoyl-Lys-Gly-His-Lys-NH₂.

## Claims

1. Dermatological composition comprising a peptide corresponding to general formula (I)
X-Gly-His-Lys-Y (I) (SEQ ID Nos. 1-2)
or a conjugate corresponding to general formula (II)
A-X-Gly-His-Lys-Y (II) (SEQ ID Nos. 3-4)
in which
A represents the radical corresponding to
- a monocarboxylic acid of general formula (III)
HOOC-R (III)
in which
R represents a linear or branched C₁-C₂₄ aliphatic radical optionally substituted with a hydroxyl group, possibly containing one or more unsaturations, advantageously from 1 to 6,
- Lipoic acid or its reduced form, dihydrolipoic acid, N-lipoyl-lysine or else retinoic acid,
X represents a chain of 1 to 3 Lys residues, that are optionally methylated,
Y represents an -OH or-NH₂ group,
the amino acids being in D, L or DL form,
in the form of enantiomers or of diastereoisomers, and also the mixtures thereof, including racemic mixtures, for use in the preventive and curative treatment of alopecia.

2. Dermatological composition for use according to Claim 1, **characterized in that** the acid of general formula (III) is an omega-3 acid chosen from α-linolenic acid, cervonic acid, timnodonic acid and pinolenic acid or else a C₁-C₂₄ aliphatic radical chosen from acetic acid, myristic acid, palmitic acid, and hydroxydecenoic and decenoic acids, and in particular trans-10-hydroxy-Δ2-decenoic acid and trans-oxo-9-decen-2-oic acid, or else an acid chosen from lipoic acid or its reduced form, dihydrolipoic acid, N-lipoyl-lysine or else retinoic acid.

3. Dermatological composition for use according to Claim 1 or 2, **characterized in that** A is chosen from lipoic acid and acetic acid.

4. Dermatological composition for use according to any one of Claims 1 to 3, **characterized in that** the conjugate is chosen from:
| | | |
|---|---|---|
| 1- | A-MeLys-Lys-Lys-Gly-His-Lys-NH₂ | (SEQ ID No. 5), |
| 2- | A-MeLys-Lys-Gly-His-Lys-NH₂ | (SEQ ID No. 6), |
| 3- | A-MeLys-Gly-His-Lys-NH₂ | (SEQ ID No. 7), |
| 4- | A-MeLys-Lys-Lys-Gly-His-Lys-OH | (SEQ-ID No. 8), |
| 5- | A-MeLys-Lys-Gly-His-Lys-OH | (SEQ ID No. 9), |
| 6- | A-MeLys-Gly-His-Lys-OH | (SEQ ID No. 10), |
| 7- | A-Lys-Lys-Gly-His-Lys-NH₂ | (SEQ ID No. 11), |
| 8- | A-Lys-Gly-His-Lys-NH₂ | (SEQ ID No. 12), |
| 9- | A-Lys-Lys-Gly-His-Lys-OH | (SEQ ID No. 13), |
| 10- | A-Lys-Gly-His-Lys-OH | (SEQ ID No. 14), |
A being an acid of general formula (III) as defined in any one of claims 1 to 3.

5. Dermatological composition for use according to any one of claims 1 to 4, **characterized in that** the peptide or peptide conjugate is chosen from:
Lipoyl-Lys-Gly-His-Lys-NH₂,
Ac-Lys-Gly-His-Lys-NH₂.

6. Dermatological composition for use according to any one of Claims 1 to 5, **characterized in that**, combined with the peptide or peptide conjugate, is a compound that improves hair regrowth, chosen from minoxidil, nicotinic acid esters, anti-inflammatory agents, retinoic acid or derivatives thereof, retinol or 5α-reductase inhibitors.

7. Dermatological composition for use according to any one of Claims 1 to 5, **characterized in that**, combined with the peptide or conjugate, is another peptide corresponding to the formula
W-Lys-Asp-Val-Z (I) (SEQ ID Nos. 15-16)
or the peptide conjugate thereof corresponding to formula (II)
A-W-Lys-Asp-Val-Z (II) (SEQ ID Nos. 17-18)
in which
A has the same definition as that given in any one of claims 1 to 3,
and W represents
Glu-Gln-Arg, Arg-Lys, Arg-Lys-Asp, Arg or a bond,
when Z represents
Tyr-Val-Gln-Leu-Tyr-NH₂, Leu-DOPA, DOPA-NH₂ or HomoPhe-NH₂,
or else W represents
Gly-Gln-Gln or Glu-Gln,
when Z represents
Tyr-Val-Gln-Leu-Tyr-NH₂, Leu-DOPA, Val-Tyr-OH, Val-Tyr-NH₂, Tyr-NH₂, Tyr-OH, DOPA-NH₂ or HomoPhe-NH₂,
in the form of enantiomers or of diastereoisomers, and also the mixtures thereof, including racemic mixtures, and the complexes with zinc which can be formed with these peptides or peptide conjugates.

8. Dermatological composition for use according to any one of Claims 1 to 7, **characterized in that** the cosmetic or dermatological composition is intended for topical application and **in that** a UVB-screening agent chosen from p-aminobenzoic acid or PABA and esters thereof, such as ethylhexyl dimethylPABA or PEG-25 PABA; cinnamates, such as ethylhexyl methoxycinnamate, isoamyl p-methoxycinnamate or octocrylene; salicylates, such as homosalate or ethylhexyl salicylate; benzimidazoles, such as phenyl-benzimidazolesulfonic acid; benzylidenecamphor derivatives, such as 4-methylbenzylidenecamphor, benzylidenecamphor, camphor benzalkonium methosulfate and polyacrylamidomethylbenzylidenecamphor; and triazines, such as ethylhexyl triazone and diethylhexyl butamido triazone, is also combined.

9. Method of cosmetic treatment for combating hair loss, comprising the application to the scalp of a composition comprising a peptide or a peptide conjugate as described in any one of claims 1 to 5, optionally combined with a compound that improves hair regrowth as defined in claims 6 and 7, and with a UVB-screening agent as defined in claim 8.

10. Peptide conjugate of formula lipoyl-Lys-Gly-tlis-Lys-NH₂.

## Patentansprüche

1. Dermatologische Zusammensetzung, enthaltend ein Peptid gemäß der allgemeinen Formel (I)
X-Gly-His-Lys-Y (I) (SEQ ID N° 1-2)
oder ein Konjugat gemäß der allgemeinen Formel (II)
A-X-Gly-His-Lys-Y (II) (SEQ ID N°3-4), worin
A den Rest darstellt, welcher
- einer Monocarbonsäure der allgemeinen Formel (III)
HOOC-R (III),
worin
R einen aliphatischen, linearen oder verzweigten, gegebenenfalls mit einer Hydroxygruppe substituierten C₁-C₂₄-Rest, welcher eine oder mehrere Unsättigungen, vorteilhafterweise 1 bis 6 Unsättigungenelbindungen, aufweisen kann, darstellt,
- Liponsäure oder deren reduzierter Form, Dihydroliponsäure, N-Lipoyl-Lysin oder Retinsäure,
entspricht,
X eine Kette mit 1 bis 3, gegebenenfalls methylierten, Lys-Resten darstellt, und
Y eine -OH- oder -NH₂-Gruppe darstellt,
wobei die Aminosäuren in D-, L- oder DL-Form vorliegen,
in Form von Enantiomeren oder Diastereoisomeren sowie deren Mischungen, einschließlich racemischer Mischungen,
zur Verwendung bei der präventiven und kurativen Behandlung von Alopezie.

2. Dermatologische Zusammensetzung zur Verwendung gemäß Anspruch 1, **dadurch gekennzeichnet, dass** die Säure der allgemeinen Formel (III) eine Omega-3-Säure ist, die aus α-Linolensäure, Cervonsäure, Timnodonsäure und Pinolensäure ausgewählt ist, oder ein aliphatischer C₁-C₂₄-Rest ist, der aus Essigsäure, Myristinsäure, Palmitinsäure, Hydroxydecensäuren und Decensäuren, insbesondere trans-10-Hydroxy-Δ2-decensäure und trans-Oxo-9-decen-2-säure, ausgewählt ist, oder eine Säure ist, die aus Liponsäure oder deren reduzierter Form, Dihydroliponsäure, N-Lipoyl-Lysin oder Retinsäure ausgewählt ist.

3. Dermatologische Zusammensetzung zur Verwendung gemäß Anspruch 1 oder 2, **dadurch gekennzeichnet, dass** A aus Liponsäure und Essigsäure ausgewählt ist.

4. Dermatologische Zusammensetzung zur Verwendung gemäß irgendeinem der Ansprüche 1 bis 3, **dadurch gekennzeichnet, dass** das Konjugat aus
1- A-MeLys-Lys-Lys-Gly-His-Lys-NH₂ (SEQ ID N°5)
2- A-MeLys-Lys-Gly-His-Lys-NH₂ (SEQ ID N°6)
3- A-MeLys-Gly-His-Lys-NH₂ (SEQ ID N°7)
4- A-MeLys-Lys-Lys-Gly-His-Lys-OH (SEQ ID N°8)
5- A-MeLys-Lys-Gly-His-Lys-OH (SEQ ID N°9)
6- A-MeLys-Gly-His-Lys-OH (SEQ ID N°10)
7- A-Lys-Lys-Gly-His-Lys-NH₂ (SEQ ID N°11)
8- A-Lys-Gly-His-Lys-NH₂ (SEQ ID N°12)
9- A-Lys-Lys-Gly-His-Lys-OH (SEQ ID N°13)
10- A-Lys-Gly-His-Lys-OH (SEQ ID N°14),
ausgewählt ist, wobei A eine Säure der allgemeinen Formel (III), wie in irgendeinem der Ansprüche 1 bis 3 definiert, ist.

5. Dermatologische Zusammensetzung zur Verwendung gemäß irgendeinem der Ansprüche 1 bis 4, **dadurch gekennzeichnet, dass** das Peptid oder Peptidkonjugat aus
Lipoyl-Lys-Gly-His-Lys-NH₂
Ac-Lys-Gly-His-Lys-NH₂
ausgewählt ist.

6. Dermatologische Zusammensetzung zur Verwendung gemäß irgendeinem der Ansprüche 1 bis 5, **dadurch gekennzeichnet, dass** man dem Peptid oder Peptidkonjugat eine das Haarwachstum verbessernde Verbindung zusetzt, die aus Minoxidil, Nikotinsäureestern, entzündungshemmenden Agenzien, Retinsäure oder deren Derivaten, Retinol oder Inhibitoren der 5α-Reduktase ausgewählt ist.

7. Dermatologische Zusammensetzung zur Verwendung gemäß irgendeinem der Ansprüche 1 bis 5, **dadurch gekennzeichnet, dass** man dem Peptid oder dem Konjugat ein weiteres Peptid gemäß der Formel
W-Lys-Asp-Val-Z (I) (SEQ ID N°15-16)
oder dessen Peptidkonjugat der Formel (II)
A-W-Lys-Asp-Val-Z (II) (SEQ ID N°17-18),
worin A wie in irgendeinem der Ansprüche 1 bis 3 definiert ist,
und W Glu-Gln-Arg, Arg-Lys, Arg-Lys-Asp, Arg oder eine Bindung darstellt, wenn Z Tyr-Val-Gln-Leu-Tyr-NH₂, Leu-DOPA, DOPA-NH₂ oder HomoPhe-NH₂ darstellt,
oder W Gly-Gln-Gln oder Glu-Gln darstellt, wenn Z Tyr-Val-Gln-Leu-Tyr-NH₂, Leu-DOPA, Val-Tyr-OH, Val-Tyr-NH₂, Tyr-NH₂, Tyr-OH, DOPA-NH₂ oder HomoPhe-NH₂ darstellt,
in Form von Enantiomeren oder Diastereoisomeren sowie deren Mischungen, einschließlich racemischer Mischungen und Komplexen mit Zink, die mit diesen Peptiden oder Peptidkonjugaten gebildet werden können, zusetzt.

8. Dermatologische Zusammensetzung zur Verwendung gemäß irgendeinem der Ansprüche 1 bis 7, **dadurch gekennzeichnet, dass** die dermatologische Zusammensetzung zur topischen Anwendung bestimmt ist, und dass außerdem ein UVB-Filter zugesetzt wird, der aus p-Aminobenzoesäure oder PABA und deren Estern, wie beispielsweise Ethylhexyldimethyl-PABA oder PEG-25-PABA; Cinnamaten, wie beispielsweise Ethylhexylmethoxycinnamat, Isoamyl-p-methoxycinnamat und Octocrylen; Salicylaten, wie beispielsweise Homosalat und Ethylhexylsalicylat; Benzimidazolen, wie beispielsweise Phenylbenzimidazolsulfonsäure; Benzylidenkampferderivaten, wie beispielsweise 4-Methylbenzylidenkampfer, Benzylidenkampfer, Kampferbenzalkoniummethosulfat und Polyacrylamidomethylbenzylidenkampfer; und Triazinen, wie beispielsweise Ethylhexyltriazon und Diethylhexylbutamidotriazon, ausgewählt ist.

9. Kosmetisches Behandlungsverfahren zur Bekämpfung von Haarausfall, welches die Anwendung einer Zusammensetzung, welche ein Peptid oder ein Peptidkonjugat, wie in irgendeinem der Ansprüche 1 bis 5 beschrieben, gegebenenfalls in Verbindung mit einer Verbindung, die das Haarwachstum verbessert, wie in den Ansprüchen 6 und 7 definiert, und einem UVB-Filter, wie in Anspruch 8 definiert, enthält, auf der Kopfhaut umfasst.

10. Peptidkonjugat der Formel Lipoyl-Lys-Gly-His-Lys-NH₂.
